# EUROPEAN PATENT SPECIFICATION

(11) **EP 3 537 976 B1**
(45) Date of publication and mention of the grant of the patent: **20.05.2020**
(21) Application number: 17797310.4
(22) Date of filing: 07.11.2017
(51) Int. Cl.: A61B 6/03, A61B 6/00, A61B 5/055, A61B 5/00, A61B 6/06

(54) **SELECTING ACQUISITION PARAMETER FOR IMAGING SYSTEM**
AUSWAHL VON AKQUISITIONSPARAMETERN FÜR BILDGEBUNGSSYSTEM
PARAMÈTRES DE SÉLECTION D'ACQUISITION POUR SYSTÈME D'IMAGERIE

(30) Priority: 10.11.2016 EP 16198086
(43) Date of publication of application: 18.09.2019
(73) Proprietor: Koninklijke Philips N.V., 5656 AG Eindhoven (NL)
(72) Inventor: SAALBACH, Axel, 5656 AE Eindhoven (NL); SENEGAS, Julien, 5656 AE Eindhoven (NL); GROTH, Alexandra, 5656AE Eindhoven (NL); JOCKEL, Sascha, Andreas, 5656 AE Eindhoven (NL); BERGTHOLDT, Martin, 5656 AE Eindhoven (NL)
(74) Representative: Zhu, Di
(86) International application number: PCT/EP2017/078461
(87) International publication number: WO 2018/087083

(56) References cited:
- WO-A1-2016/073841
- US-A1- 2015 199 478

## Description

### FIELD OF THE INVENTION

The invention relates to a system and a method for selecting an acquisition parameter for an imaging system. The invention further relates to a workstation and imaging system comprising the system, and to a computer readable medium comprising instructions for causing a processor system to perform the methods.

### BACKGROUND OF THE INVENTION

A common problem in medical imaging is the selection of appropriate acquisition parameters by a technician or other user. Such acquisition parameters may at least in part define an imaging configuration of an imaging system, and are typically patient-specific. For example, it is frequently difficult for a technician to configure the collimation in an X-ray imaging system to ensure, on the one hand, a sufficient coverage of the target anatomy in order to ensure the diagnostic value of the acquired image, e.g., by avoiding the need for re-takes, while on the other hand, minimizing over-collimation in order to ensure the safety of the patient, e.g., by avoiding unnecessary exposure to ionizing radiation.

Other acquisition parameters of an X-ray imaging system of which the selection is patient-specific include, but are not limited to, parameters specifying the tube voltage (often measured in kV) and the tube current (in mA) of the X-ray tube. Typically, the technician is guided in the selection of the acquisition parameters by medical guidelines. However, in practice, the successful selection of acquisition parameters may depend at least in part on the expertise and experience of the operator, and may involve trial and error. Disadvantageously, such trial and error may result in increased radiation exposure, e.g., in X-ray imaging, but also longer examination time, insufficient image quality, etc.

It is noted that the above also applies to other imaging modalities, including but not limited to Magnetic Resonance imaging and Ultrasound, mutatis mutandis.

WO2016001135 describes a method which comprises configuring an X-ray imaging system. The method is said to comprise the steps of obtaining one or more depth images from one or more depth cameras, with said one or more depth cameras covering at least an area covered by an X-ray bundle of an X-ray source of said X-ray imaging system. In an example relating to taking an X-ray image of a knee, it is said that from the depth image, the knee is identified and located in the depth image, e.g., using image recognition software which is available on the market for the processing of depth images. The system further retrieves a desired position of the knee with respect to the depth image. It is said that the difference between the actual position and the desired position then determines a correction for the configuration of the X-ray imaging system. With the geometric relation between the depth cameras and position of the X-ray source being known, e.g., based on calibration or machine learning techniques such as adaptive filtering, the correction may then be translated into the actual position configuration of the X-ray system.

As such, WO2016001135 detects an object in the depth image while, separately thereof, obtaining the geometric relation between the depth cameras and position of parts of the X-ray source. Both are then combined to configure the position of the X-ray system so as to obtain a desired position of the object in the depth image.

Disadvantageously, not all relevant landmarks may be reliably detected in depth images. In addition, by only using detected landmarks to determinate of the position configuration, other potentially useful information in the depth image is not used. WO2016/073841 A1 discloses a system for selecting an acquisition parameter using a machine learning algorithm for landmark detection in a depth-related map.

### SUMMARY OF THE INVENTION

It would be advantageous to obtain a system and method for selecting an acquisition parameter for an imaging system which addresses one or more of the above problems, for example, by not solely relying on landmark detection.

A first aspect of the invention provides a system for selecting an acquisition parameter for an imaging system, wherein the acquisition parameter at least in part defines an imaging configuration of the imaging system during an imaging procedure with a patient, comprising:
- a camera data interface configured to access a depth-related map which is generated on the basis of sensor data from a camera system, wherein the camera system has a field of view which includes at least part of a field of view of the imaging system, wherein the sensor data is obtained before the imaging procedure with the patient and indicative of a distance that parts of the patient's exterior have towards the camera system;
- a memory comprising instruction data representing a set of instructions;
- a processor configured to communicate with the camera data interface and the memory and to execute the set of instructions, wherein the set of instructions, when executed by the processor, cause the processor to apply a machine learning algorithm to the depth-related map to identify the acquisition parameter, wherein:
   - the machine learning algorithm is represented by algorithm data which is stored in the memory and accessed by the processor, and
   - the machine learning algorithm is trained using training data comprising sets of i) an example depth-related map and ii) as prediction value, an example acquisition parameter, wherein the example acquisition parameter represents a selection by a human operator in a previous imaging procedure with a previous patient, wherein the example depth-related map is generated on the basis of sensor data obtained by a same or similar type of camera system during the previous imaging procedure; and
- an output interface configured to output the acquisition parameter to be used by the imaging system.

A further aspect of the invention provides a workstation or imaging system comprising the system.

A further aspect of the invention provides a computer implemented method for selecting an acquisition parameter for an imaging system, wherein the acquisition parameter at least in part defines an imaging configuration of the imaging system during an imaging procedure with a patient, comprising:
- accessing a depth-related map which is generated on the basis of sensor data from a camera system, wherein the camera system has a field of view which includes at least part of a field of view of the imaging system, wherein the sensor data is obtained before the imaging procedure with the patient and indicative of a distance that different parts of the patient's exterior have towards the camera system;
- applying a machine learning algorithm to the depth-related map to identify the acquisition parameter, wherein:
   - the machine learning algorithm is represented by algorithm data which is stored in the memory and accessed by the processor, and
   - the machine learning algorithm is trained using training data comprising sets of i) an example depth-related map and ii) as prediction value, an example acquisition, wherein the example acquisition parameter represents a selection by a human operator for use in a previous imaging procedure with a previous patient, wherein the example depth-related map is generated on the basis of sensor data obtained by a previous camera system during the previous imaging procedure; and
- outputting the acquisition parameter to be used by the imaging system.

A further aspect of the invention provides a computer readable medium comprising transitory or non-transitory data representing instructions arranged to cause a processor system to perform the method.

The above measures involve obtaining a depth-related map of the exterior of the patient before the imaging procedure, e.g., in the form of a depth or disparity image. As such, the depth-related map may provide information on physical attributes of the patient's exterior. The depth-related map may be acquired when the patient is positioned for the imaging procedure, e.g., by lying or standing within the field of view of the imaging system. The depth-related map does not necessarily have to include the whole exterior of the patient; it may relate to only part of the exterior. A measured depth may locally vary. One simple example is that a neck of the patient will typically be recessed with respect to face and thus be further away from the camera. Similarly, in case of obese patient, a waist may protrude and thus be closer to camera. The above measures further provide a processor configured by way of a set of instructions to apply a machine learning algorithm to the depth-related map, which has previously been trained on example depth-related maps of previous patients and human-selected parameters for the previous patients. It is noted that with the word 'previous' is meant to denote 'past', so not limited to immediately preceding.

The inventors have recognized that depth images and other depth-related maps may provide various information about the patient's exterior which is valuable in determining acquisition parameters. Such information is not used when the depth-related map is solely used for detecting landmarks. For example, various patient attributes may be visible in the depth image, or at least an indication may be obtained, including but not limited to the patient's body mass (weight), body type, sex, fitness level. These patient attributes may influence acquisition parameter to be used in the imaging procedure. However, designing heuristics is very difficult as the relations may be complex and in particular non-linear.

By using machine learning for the depth-related map, which has been trained on previous patients and manually selected parameters, all or at least more of the available information of the depth-related map may be taken into account. By training the machine learning algorithm, the system is able to automatically model the relation between depth and the acquisition parameter. Thus there is no need to solely rely on landmark detection (which may not always be used, e.g., if a landmark is not visible). In fact, determined landmarks may be considered an artificial intermediate representation, which involves assigning coordinates to detected objects and then further only using these coordinates. The proposed system is configure to apply machine learning to all of the depth-related map which result a more 'holistic' approach which does not need this artificial intermediate representation.

Optionally, the system comprises a patient data interface configured to access non-image patient data of the patient, and the training data further comprises, for a given set of the training data, example non-image patient data which is of a same or similar type as the non-image patient data of the patient; and wherein the set of instructions, when executed by the processor, cause the processor to use the non-image patient data as additional input to the machine learning algorithm. The non-image data may comprise complementary information to the depth-related data. For example, the size of the lung is known to be correlated to the patient weight, age and sex and may be influenced by certain diseases like COPD. By additionally taking into account such non-image data, the system is able to better model the relation between patient and acquisition parameter to be used in the imaging procedure.

Optionally, the patient data interface is configured to access the non-image patient data from an electronic health record of the patient.

Optionally, the non-image patient data of the patient comprises at least one of:
- a weight of the patient;
- an age of the patient;
- a sex of the patient;
- a quantification of a fitness level of the patient;
- a disease diagnosis associated with the patient;
- a medication record associated with the patient, and
- a vital parameter record associated with the patient.

Optionally, the training data further comprises, for a given set of the training data, example geometry data which is indicative of a previous relative geometry between camera system and imaging system during the previous imaging procedure, and the set of instructions, when executed by the processor, cause the processor use a current relative geometry between the camera system and the imaging system in the imaging procedure as additional input to the machine learning algorithm. The machine learning algorithm thus differentiates between different relative geometries between camera system and imaging system during the training as well as subsequent use. Here, the term 'relative geometry' may refer to a geometric relationship between the field of view of the camera system and the field of view of the imaging system. Differences in relative geometry may exist due to, e.g., differences in mounting of the camera system (e.g., to the imaging system or standing alone), or differences in field of view of the camera system and/or the imaging system itself. In this way, it may be prevented that training data which is obtained within the context of a particular relative geometry is used during imaging procedures where the camera system and the imaging system have a different relative geometry. It is noted that, alternatively, it may be ensured that the camera system and imaging system have a same or similar relative geometry during training and subsequent use of the machine learning algorithm.

Optionally, the example depth-related map of a given set of the training data is generated on the basis of a previous relative geometry between camera system and imaging system, and the set of instructions, when executed by the processor, cause the processor to:
- determine a deviation between the previous relative geometry and a current relative geometry between the camera system and the imaging system in the imaging procedure;
- if the deviation exists or exceeds a threshold, process the depth-related map to compensate for the deviation before said applying of the machine learning algorithm.

By processing the depth-related map in the described manner, standardized input data may be provided to the machine learning algorithm. For example, the deviation may result in a change in field of view of the camera system. Image processing techniques from the fields of perspective image correction may be used to compensate for such differences in field of view. Various other techniques are known as well and may be advantageously used. In this respect, it is noted that the depth-related map may be considered an image and thus processed by such image processing techniques.

Optionally, the camera data interface is further configured to access image data acquired by the camera system, wherein the image data shows the patient's exterior, and wherein:
- the training data further comprises, for a given set of the training data, example image data acquired by the previous camera system which shows the previous patient's exterior during the previous imaging procedure; and
- the set of instructions, when executed by the processor, cause the processor use the image data as additional input to the machine learning algorithm.

In addition to the depth-related map, also an image of the patient's exterior may be used as input. The image may be a 'normal' visible light image, e.g., as obtained by a camera having a RGB sensor for visible light. The field of view provided by the image may be the same or at least overlap with the field of view provided by the depth-related map. By additionally taking into account such image data, the system is able to better model the relation between patient and acquisition parameter to be used in the imaging procedure.

Optionally, the system comprises the camera system, wherein the camera system comprises at least one of a time-of-flight camera, a light detection and ranging (LiDAR) camera, a laser detection and ranging (LaDAR) camera, a stereo camera, or two cameras arranged as a stereo camera; and a projector configured to project a known pattern onto the patient's exterior, thereby yielding a deformed pattern, and a camera configured to record the deformed pattern.

Optionally, the imaging system is a Magnetic Resonance imaging system, and wherein the acquisition parameter is one of:
- a parameter specifying the positioning of the patient with respect to the Magnetic Resonance imaging system, such as a reference point for a region to be moved to the magnetic isocenter of the Magnetic Resonance imaging system;
- a geometry acquisition parameter, such as a center, orientation, and/or size of the imaging slices to be acquired;
- a selection parameter for a pre-set protocol;
- a SENSE factor; and
- a SENSE direction.

Here, the term 'SENSE' refers to sensitivity encoding, which is described in the article *"*SENSE: Sensitivity Encoding for Fast MRI", by Klaas P. Pruessmann et al., Magnetic Resonance in Medicine 42:952-962 (1999). The SENSE factor is the undersampling factor. The SENSE direction is the direction of sensitivity encoding.

Optionally, the imaging system is an X-ray imaging system, and wherein the acquisition parameter is one of:
- a tube voltage;
- a tube current;
- a grid;
- a collimation window;
- a geometry parameter of a collimator, such as a detector height, tilt of a tube, rotation of the collimator.

Optionally, the imaging system is a Computed Tomography imaging system, and wherein the acquisition parameter is one of:
- a power supply level;
- a tube current,
- a dose modulation;
- a scan planning parameter, such as a start and end position of a table for a localizer;
- a reconstruction parameter, such as a filter type to be used.

Optionally, the machine learning algorithm is a convolutional neural network. Alternatively, the machine learning algorithm may comprise Support Vector Machines, Decision Trees, Nearest neighbor techniques, or Ensemble methods, e.g., combined with feature detectors and/or feature descriptors known in the field of feature detection, such as, but not limited to, SURF, SIFT, Brief, Brisk or techniques like Eigenimages.

It will be appreciated by those skilled in the art that two or more of the above-mentioned embodiments, implementations, and/or optional aspects of the invention may be combined in any way deemed useful.

Modifications and variations of the workstation, the imaging system, the computer-implemented method, and/or the computer program product, which correspond to the described modifications and variations of the system, can be carried out by a person skilled in the art on the basis of the present description.

A person skilled in the art will appreciate that the system and computer-implemented method may be applied to multi-dimensional image data, e.g., two-dimensional (2D), three-dimensional (3D) or four-dimensional (4D) images, acquired by various acquisition modalities such as, but not limited to, standard X-ray Imaging, Computed Tomography (CT), Magnetic Resonance Imaging (MRI), Ultrasound (US), Positron Emission Tomography (PET), Single Photon Emission Computed Tomography (SPECT), and Nuclear Medicine (NM).

### BRIEF DESCRIPTION OF THE DRAWINGS

These and other aspects of the invention will be apparent from and elucidated further with reference to the embodiments described by way of example in the following description and with reference to the accompanying drawings, in which
Fig. 1 schematically shows a system which is configured for selecting one or more acquisition parameters for an imaging system;
Fig. 2 and 3 illustrate depth maps obtained from different patients;
Fig. 4 schematically shows a convolution neural network which is used in a machine learning algorithm according to an embodiment of the invention;
Fig. 5 shows another embodiment of a system which is configured for selecting one or more acquisition parameters for an imaging system;
Fig. 6 shows a flow chart of a method for selecting an acquisition parameter for an imaging system according to an embodiment; and
Fig. 7 shows a computer readable medium comprising instructions for causing a processor system to perform the method.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### LIST OF REFERENCE NUMBERS

The following list of reference numbers is provided for facilitating the interpretation of the drawings and shall not be construed as limiting the claims.
- 10: patient
- 11: support
- 12: room
- 15: imaging system
- 16: field of view of the imaging system
- 18: camera system
- 19: field of view of the camera system
- 58: first camera
- 59: second camera
- 60: patient information interface
- 62: patient information database
- 100: system for selecting an acquisition parameter
- 102: depth map of first patient
- 103: depth map of second patient
- 120: camera data interface
- 122: camera or depth data
- 140: memory
- 142: internal data communication
- 160: processor
- 162: output representing acquisition parameter
- 170: output interface
- 200: convolutional neural network
- 201: first layer
- 202: second layer
- 203: third layer
- 204: fourth layer
- 205: fifth layer
- 206: sixth layer
- 207: output neuron
- 400: method for selecting an acquisition parameter for an imaging system
- 410: accessing depth-related map
- 420: applying machine learning algorithm
- 430: outputting acquisition parameter
- 500: computer readable medium
- 510: non-transitory data representing instructions

### DETAILED DESCRIPTION OF EMBODIMENTS

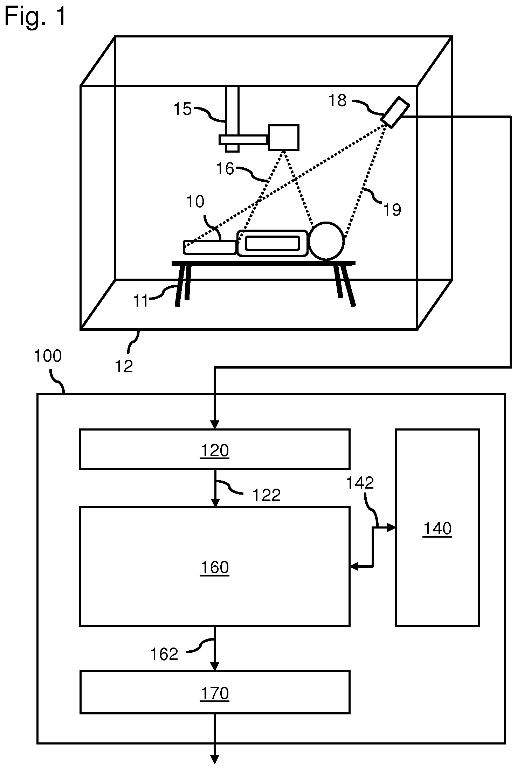

Fig. 1 schematically shows a system 100 which is configured for selecting an acquisition parameter for an imaging system, wherein the acquisition parameter at least in part defines an imaging configuration of the imaging system during an imaging procedure with a patient. Fig. 1 schematically shows the patient 10 lying on a support 11 in a room 12. An X-ray imaging system 15 is arranged above the support 11, the X-ray imaging system 15 having a field of view 16 which is also referred to as activation area 16. A camera system 18 is arranged above the support 11 so as to be able to make images of the patient 10.

It is noted that the X-ray imaging system 15 does not have to be positioned above the patient. For example, in a near controlled fluoroscopy system, the X-ray tube may be positioned underneath the support and the imager / receptor above the patient. In other embodiments the X-ray imaging system 15 may be arranged in front or behind the patient.

The camera 18 has a field of view 19 which at least partly overlaps the field of view 16 of the X-ray imaging system 15. In the example of Fig. 1, the camera system 18 is remote from the X-ray imaging system 15. Alternatively the camera system 18 may be affixed to the X-ray imaging system 15, e.g., to a C-arm of an X-ray imaging system.

The system 100 comprises a camera data interface 120 configured to access a depth-related map on the basis of sensor data obtained from the camera system 18. The sensor data is obtained before the imaging procedure with the patient 10 and indicative of a distance that parts of the patient's exterior have towards the camera system 18. The camera system 18 may directly provide the depth-related map to the system 100. Alternatively, the camera system 18 may provide the sensor data to the system 100, with the sensor data being indicative of, but not directly representing a depth-related map. A non-limiting example of such sensor data is the image data of stereo images. In this and similar cases, a depth-related map may be estimated from the sensor data using known techniques, e.g., from the field of depth- and/or disparity estimation. The depth-related map may be estimated by the system 100 itself, e.g., by a depth estimation processor (not shown in Fig. 1) or by the camera data interface 120 itself, or by an external entity. As explained further onwards, the depth-related map may be a depth map but also a disparity map or other type of depth-related map.

In an embodiment, the camera system 18 may be separate from the system 100. However, the system 100 may also comprise the camera system 18.

In general, the camera system 18 may comprises at least one of: a time-of-flight camera, a stereo camera, two cameras arranged as a stereo camera, and a projector configured to project a known pattern onto the patient's exterior, thereby yielding a deformed pattern, and a camera configured to record the deformed pattern.

The camera data interface 120 may take various forms, such as a network interface to a local area network, but also a video interface, e.g., HDMI, etc.

The system 100 further comprises a processor 160 configured to internally communicate with the camera data interface 120 via data communication 122, as well as a memory 140 accessible by the processor 160 via data communication 142.

The memory 140 may comprise instruction data representing a set of instructions which configures the processor 160 to, during operation of the system 100, communicate with the camera data interface 120 and the memory 140 and to apply a machine learning algorithm to the depth-related map to identify the acquisition parameter.

The machine learning algorithm may be represented by algorithm data which is stored in the memory 140 and accessed by the processor 160. Furthermore, the machine learning algorithm may trained during a training phase using training data comprising sets of i) an example depth-related map and ii) as prediction value, an example acquisition parameter, wherein the example acquisition parameter represents a selection by a human operator in a previous imaging procedure with a previous patient, wherein the example depth-related map is generated on the basis of sensor data obtained by a same or similar type of camera system 18 during the previous imaging procedure.

Fig. 1 further shows the system 100 comprising an output interface 170. In the example of Fig. 1, the output interface 170 is configured to send the output representing the acquisition parameter 162 externally. For example, the output interface 170 may be configured to provide the output 162 to an imaging system for use in the imaging procedure, e.g., to a workstation which is part thereof and which controls the X-ray imaging system 15. Alternatively, the system 100 may be part of an imaging system such as the X-ray imaging system 15; in this case, the output interface 170 may be an internal output interface.

The system 100 may be embodied as, or in, a device or apparatus, such as a server, workstation, imaging system or mobile device. The device or apparatus may comprise one or more microprocessors or computer processors which execute appropriate software. The processor of the system may be embodied by one or more of these processors. The software may have been downloaded and/or stored in a corresponding memory, e.g., a volatile memory such as RAM or a non-volatile memory such as Flash. The software may comprise instructions configuring the one or more processors to perform the functions described with reference to the processor of the system. Alternatively, the functional units of the system, e.g., the camera data interface, an optional patient data interface and the processor, may be implemented in the device or apparatus in the form of programmable logic, e.g., as a Field-Programmable Gate Array (FPGA). The camera data interface and the patient data interface may be implemented by respective interfaces of the device or apparatus. In general, each functional unit of the system may be implemented in the form of a circuit. It is noted that the system 100 may also be implemented in a distributed manner, e.g., involving different devices or apparatuses. For example, the distribution may be in accordance with a client-server model, e.g., using a server and a thin-client PACS workstation.

Publication WO 2014033614 A1, which is hereby incorporated by reference, describes the use of spatial depth information in an apparatus for automatically or semiautomatically controlling a collimator of an x-ray imager to collimate imager's x-ray beam and adjusting an alignment of the x-ray imager in respect of an object. The collimation and alignment operation is based on 3D image data of the object to be imaged. The 3D image data is acquired by a sensor. The 3D image data describes a shape in 3D of the object and anatomic landmarks are derived therefrom to define a collimation window for a region of interest. So in WO 2014033614 A1 landmarks are needed to find the proper acquisition parameter being the collimation window. No use is made of machine learning algorithms to be able to determine acquisition parameter(s) in an empirical manner as is described in the present application. However, the acquisition of the 3D image data is incorporated by reference as representing possible embodiments of the acquisition of the depth-related map.

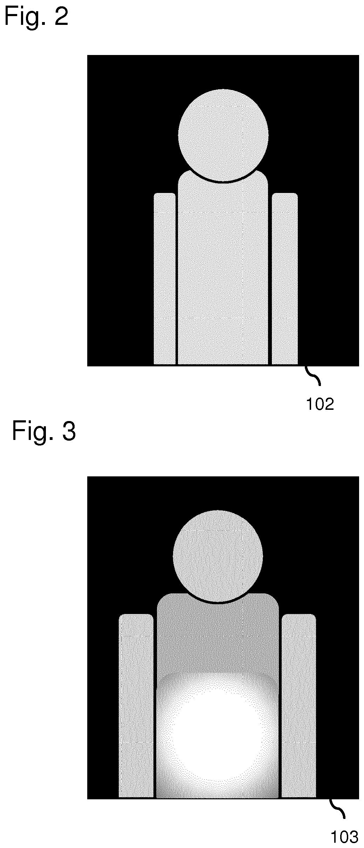

Fig. 2 and 3 illustrate depth images from different patients (which may also be referred to as 'depth maps' throughout this specification). Fig. 2 show a depth image 102 of a 'normal' patient, whereas Fig. 3 shows a depth image 103 of an obese patient. In Fig. 2 and 3 depth is indicated by a grey scale with black indicating a larger distance to the camera and white being nearer to the camera. As can be seen from Fig. 3, the belly of the obese patient protrudes as can be seen by the belly being brighter in the depth image 103. Such patient attributes, e.g., the physical state or fitness of a patient, will not be taken into account when only looking for landmarks in depth images, but are used by the system according to the invention as will be explained in more detail below.

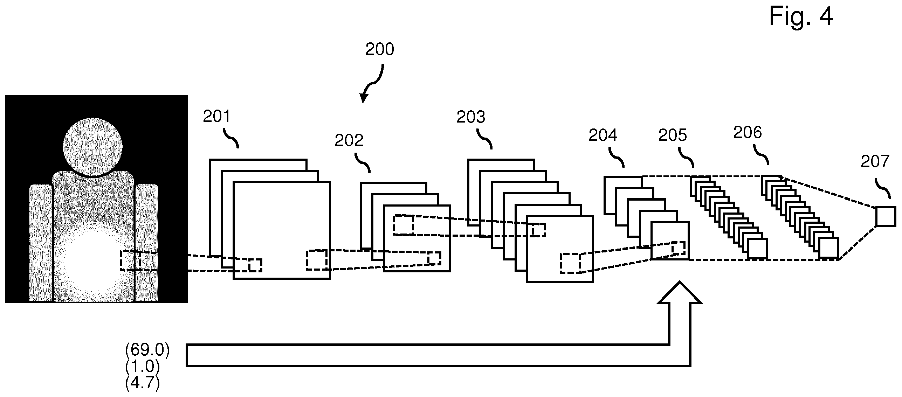

Fig. 4 schematically shows a Convolution Neural Network (CNN) 200 which may be used to predict the acquisition parameters. Unlike a regular Neural Networks (which consists of a sequence of fully connected layers of artificial neurons), a CNN consist of a set of different layers that are locally connected and a set of layers that are fully connected. In the embodiment of Fig. 4, the first set of layers comprises a convolutional layer 201, a pooling layer 202, a further convolution layer 203 and a further pooling layer 204. The convolutional layers 201, 203 consist of a set of learnable filters (which are spatially constrained and shared within the layer), while the pooling layers 202, 204 perform a non-linear down sampling (and reduces the amount of free parameters). The further pooling layer 204 is connected to a first fully connected layer 205 which is connected to a second fully connected layer 206. An output neuron 207 is fully connected to the second fully connected layer 206. A value of the output neuron, also referred to as prediction value, may be indicative for the acquisition parameter for the imaging system.

Considering the CNN architecture of Fig. 4, e.g., using convolutional and pooling layers as well as fully connected layers, the first part (layers 201-204) may be considered to basically perform an image feature extraction step, while the latter (layers 205, 206, 207) may be considered to basically perform the classification or regression task.

In order to include further patient relevant information, non-image data may be directly inserted into the first fully connected layer, see layer 205. It is noted that the number of layers and types of layers in the CNN 200 may vary depending on the learning algorithm. It is noted that the last layers of the CNN 200 are shown to be fully connected in Fig. 4. However, it should be noted that the layers 205, 206 may alternatively be partly connected depending on the particular machine learning algorithm used.

It is noted there may be more than one output neuron. For example, the CNN 200 may be trained to predict more than one acquisition parameter at a time. Also, the acquisition parameter may be coded in the network, e.g., as a categorical parameter, in which case several output neurons may be needed to code one value for the acquisition parameter. For example, the value of the acquisition parameter may be binary encoded.

While conventional CNNs have been employed mainly for image classification or for object detection (e.g., by evaluating the CNN at different positions in the image), the CNN 200 may be used as a regression algorithm, similar to YOLO as described in the article *"*You Only Look Once: Unified, Real-Time Object Detection" by J. Redmon et al., 2015. YOLO was designed to predict bounding boxes and class probabilities (e.g., x ,y, width, height, probability) for an object of interest in an RGB image.

In an embodiment, such a regression algorithm may be used in order to predict the acquisition parameter(s) for the imaging system. One application could be, e.g., the prediction of the collimation window settings for the current patient. This may result in, e.g., x, y, width and height setting of the collimation window. In general, the base technique described in the YOLO publication may be extended to provide the claimed prediction of 'arbitrary' acquisitions parameters, as well as combined with input of non-image data.

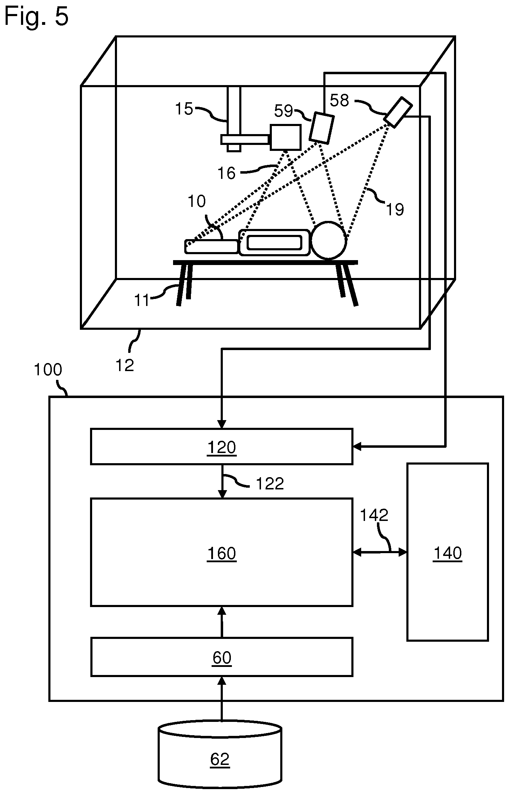

In the example of Fig. 4, a training sample is fed into a CNN 200 which sample comprises a depth-related map together with (optional) non-image data. The non-image data may comprise a length of the patient, an age of the patient, a weight of the patient or any other non-image data possibly useful to train the CNN, which will be further discussed in this specification with reference to Fig. 5. As can be seen from Fig. 4, the non-image data may be used as input for later (e.g., fully connected) layers in the network.

In an embodiment, the depth-related map may be produced by an RGB-D camera. By using the CNN 200 of Fig. 4 or a similar machine learning algorithm, the system 100 of Fig. 1 may be configured to derive information the RGB-D image which goes beyond landmarks. For example, the RGB-D image may provide information on a difference in height of the belly of the patient, which may be indicative of the patient's general fitness and thus relevant for the collimation settings, the exposure time settings, the tube voltage settings, the focal spot size settings, and selection of the X-ray sensitive area, etc.

In a further embodiment, RGB-D images, or in general depth-related maps, may be used as training data for the learning algorithm together with corresponding annotated X-Ray images. By using annotated X-Ray images, the system 100 may be trained to predict the size and position of an anatomy of interest such as, e.g., a lung of the patient. Different scenarios are possible. For example, one could predict the position of the lung in the RGB-D image (which can then be mapped into system settings given the relative geometry between the camera system and the X-Ray device). Alternatively one could predict the position of the lung in the detector coordinate system, e.g., the position relative to the camera system 18 or imaging system 15. Once the position of the lung of the patient is predicted, proper collimation settings for the X-ray imaging system can be determined so to allow for an optimal imaging of the lung, or in general of the anatomy of interest.

It is noted that the camera system 18 may provide images or a video, e.g., a stream of video images. The video may be a continuous video stream or an interval video stream. The machine learning algorithm may use individual frames of the video as input. Alternatively, multiple frames may be together used as input to the algorithm.

In a further embodiment, the camera system comprises two cameras, e.g., as shown in Fig. 5 in which the room 12 of Fig. 1 now comprises a first camera 58 and a second camera 59. Fig. 5 further resembles Fig. 1, except for a patient data interface 60 and an external patient information database 62, which will be discussed further onwards.

The first camera 58 and second camera 59 may be stereo cameras arranged to provide stereo content. In this case depth information may be provided by the differences between a left and a right image signal of the stereo content. The depth-related map may then be estimated from such stereo content using known techniques known as 'depth estimation'.

Alternatively, the first camera 58 may be a time-of-flight (ToF) camera and the second camera 59 may be a 'normal' visible light camera. The depth information produced by the ToF camera 58 may be provided explicitly in the content received by the processor 160. For example, in content encoded in the so-termed 'image+depth' format, the depth information is provided by a depth signal comprising depth values which are indicative of distances that objects within the 2D image signal have towards a camera or viewer. The depth may be obtained from the ToF camera 58, and the image from the normal camera 59.

In a further embodiment, the camera system may comprise a projector 58 configured to project a known pattern onto the patient's exterior, thereby yielding a deformed pattern, and a camera 59 configured to record the deformed pattern. The deformed pattern may be used by the processor 160 or other entity to create a depth-related map.

In yet another example, the camera system may comprise a light detection and ranging (LiDAR) camera and/or a laser detection and ranging (LaDAR) camera.

It is noted that the depth-related map may be an image-like arrangement of depth-related values. As such, the term 'depth-related map' may be understood as a reference to a depth-related image, and vice versa. The depth-related map may comprise depth values. Alternatively, the depth-related map may comprise disparity values or parallactic shift values. Disparity values and parallactic shift values have an approximately inverse relation to depth values, but are thus still representative of a 'depth', e.g., the distances that objects have towards a camera, or viewer or display but do not directly correspond to said distances. Methods for conversion between all of the above types of depth values are known per se.

The system 100 shown in Fig. 5 further comprises an optional patient data interface 60 arranged to receive patient data from the aforementioned patient information database 62. The patient information database 62 may comprise non-image patient data of several other patients which may be used by the processor 160 to train the machine learning algorithm. An example of such non-image patient data may be an electronic health record of the patients. The database 62 may also comprise values of associated acquisition parameters which may be used as prediction value during the training phase. Alternatively, such acquisition parameters may be retrieved from a different database (not shown).

It will be appreciated that the described system may have several advantages compared to landmark based approaches. It may provide for a generic framework for the prediction of scan parameters which is applicable for different examinations as well as modalities. Furthermore, it may provide an end-to-end approach which directly allows for the prediction of the required output without the need of landmark detection or a separate statistical model for the prediction of "internal" anatomical structures. Also it can easily be optimized by integrating RGB-D and medical image data. The described system may leverage the entire image information (not only landmarks) which potentially allows for a higher accuracy (it may "see" whether a patient is small or large, obese or athletic - which is related to the expected lung size). The system may easily integrate non-image information, like patient age or sex, which may facilitate the prediction process. Finally, it may allow for online learning at the clinical site, e.g., to re-train the system onsite.

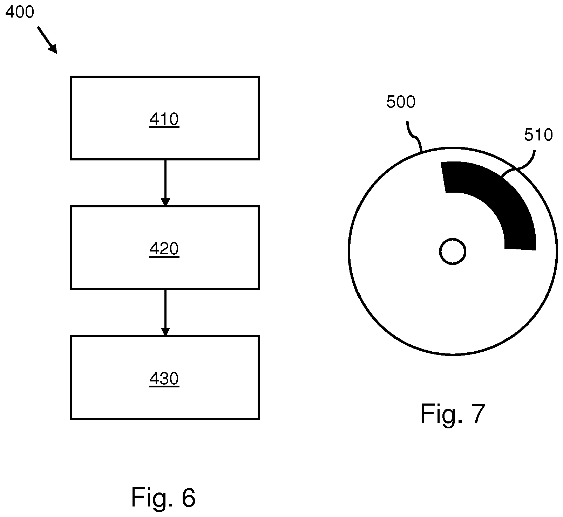

Fig. 6 shows a method 400 for selecting an acquisition parameter for an imaging system according to an embodiment. The method 400 may, but does not need to, correspond to an operation of the system 100 as described with reference to Fig. 1 and others.

The method 400 may comprise, in an operation titled "ACCESSING DEPTH-RELATED MAP", accessing 410 a depth-related map which is generated on the basis of sensor data from a camera system, wherein the camera system has a field of view which includes at least part of a field of view of the imaging system, wherein the sensor data is obtained before the imaging procedure with the patient and indicative of a distance that different parts of the patient's exterior have towards the camera system. The method 400 may further comprise, in an operation titled "APPLYING MACHINE LEARNING ALGORITHM", applying 420 a machine learning algorithm to the depth-related map to identify the acquisition parameter. The machine learning algorithm is represented by algorithm data which is stored in the memory and accessed by the processor, and the machine learning algorithm is trained using training data comprising sets of i) an example depth-related map and ii) as prediction value, an example acquisition, wherein the example acquisition parameter represents a selection by a human operator for use in a previous imaging procedure with a previous patient, wherein the example depth-related map is generated on the basis of sensor data obtained by a previous camera system during the previous imaging procedure. The method 400 may further comprise in an operation titled "OUTPUTTING ACQUISITION PARAMETER", outputting 430 the acquisition parameter to be used by the imaging system.

It will be appreciated that the above operation may be performed in any suitable order, e.g., consecutively, simultaneously, or a combination thereof, subject to, where applicable, a particular order being necessitated, e.g., by input/output relations.

The method 400 may be implemented on a computer as a computer implemented method, as dedicated hardware, or as a combination of both. As also illustrated in Fig. 7, instructions for the computer, e.g., executable code, may be stored on a computer readable medium 500, e.g., in the form of a series 510 of machine readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, servers, online software, etc. Fig. 7 shows an optical disc 500 storing non-transitory data 510 representing the instructions.

It will be appreciated that the invention also applies to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other. An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

## Claims

1. A system (100) for selecting an acquisition parameter for an imaging system (15), wherein the acquisition parameter at least in part defines an imaging configuration of the imaging system during an imaging procedure with a patient, comprising:
- a camera data interface (120) configured to access a depth-related map which is generated on the basis of sensor data from a camera system, wherein the camera system has a field of view which includes at least part of a field of view of the imaging system, wherein the sensor data is obtained before the imaging procedure with the patient and indicative of a distance that parts of the patient's exterior have towards the camera system;
- a memory (140) comprising instruction data representing a set of instructions;
- a processor (160) configured to communicate with the camera data interface (120) and the memory (160) and to execute the set of instructions, wherein the set of instructions, when executed by the processor, cause the processor to apply a machine learning algorithm to the depth-related map to identify the acquisition parameter, wherein:
- the machine learning algorithm is represented by algorithm data which is stored in the memory and accessed by the processor, and
- the machine learning algorithm is trained using training data comprising sets of i) an example depth-related map and ii) as prediction value, an example acquisition parameter, wherein the example acquisition parameter represents a selection by a human operator in a previous imaging procedure with a previous patient, wherein the example depth-related map is generated on the basis of sensor data obtained by a same or similar type of camera system during the previous imaging procedure; and
- an output interface (170) configured to output the acquisition parameter to be used by the imaging system.

2. The system (100) according to claim 1, further comprising a patient data interface (60) configured to access non-image patient data of the patient, and wherein:
- the training data further comprises, for a given set of the training data, example non-image patient data which is of a same or similar type as the non-image patient data of the patient; and
- the set of instructions, when executed by the processor (160), cause the processor (160) to use the non-image patient data as additional input to the machine learning algorithm.

3. The system (100) according to claim 2, wherein the patient data interface (60) is configured to access the non-image patient data from an electronic health record of the patient.

4. The system (100) according to claim 2 or 3, wherein the non-image patient data of the patient comprises at least one of:
- a weight of the patient;
- an age of the patient;
- a sex of the patient;
- a quantification of a fitness level of the patient;
- a disease diagnosis associated with the patient;
- a medication record associated with the patient; and
- a vital parameter record associated with the patient.

5. The system (100) according to any one of the above claims, wherein:
- the training data further comprises, for a given set of the training data, example geometry data which is indicative of a previous relative geometry between the camera system (18) and the imaging system (15) during the previous imaging procedure; and
- the set of instructions, when executed by the processor (160), cause the processor (160) to use a current relative geometry between the camera system (18) and the imaging system (15) in the imaging procedure as additional input to the machine learning algorithm.

6. The system (100) according to any one of claim 1 to 4, wherein:
- the example depth-related map of a given set of the training data is generated on the basis of a previous relative geometry between camera system (18) and imaging system (15);
- the set of instructions, when executed by the processor (160), cause the processor (160) to:
- determine a deviation between the previous relative geometry and a current relative geometry between the camera system (18) and the imaging system (15) in the imaging procedure;
- if the deviation exists or exceeds a threshold, process the depth-related map to compensate for the deviation before said applying of the machine learning algorithm.

7. The system (100) according to any one of the above claims, wherein:
- the camera data interface (120) is further configured to access image data acquired by the camera system (18), wherein the image data shows the patient's exterior;
- the training data further comprises, for a given set of the training data, example image data acquired by the previous camera system (18) which shows the previous patient's exterior during the previous imaging procedure; and
- the set of instructions, when executed by the processor (160), cause the processor (160) to use the image data as additional input to the machine learning algorithm.

8. The system (100) according to any one of the above claims, wherein the system (100) comprises the camera system (18), and wherein the camera system (18) comprises at least one of:
- a time-of-flight camera;
- a light detection and ranging (LiDAR) camera;
- a laser detection and ranging (LaDAR) camera;
- a stereo camera, or two cameras arranged as a stereo camera; and
- a projector configured to project a known pattern onto the patient's exterior, thereby yielding a deformed pattern, and a camera configured to record the deformed pattern.

9. The system (100) according to any one of the above claims, wherein the imaging system (15) is a Magnetic Resonance imaging system, and wherein the acquisition parameter is one of:
- a parameter specifying the positioning of the patient with respect to the Magnetic Resonance imaging system;
- a geometry acquisition parameter;
- a selection parameter for a pre-set protocol;
- a SENSE factor;
- a SENSE direction.

10. The system (100) according to any one of the above claims, wherein the imaging system (15) is an X-ray imaging system, and wherein the acquisition parameter is one of:
- a tube voltage;
- a tube current;
- a grid;
- a collimation window; and
- a geometry parameter of a collimator.

11. The system (100) according to any one of the above claims, wherein the imaging system (15) is a Computed Tomography imaging system, and wherein the acquisition parameter is one of:
- a power supply level;
- a tube current,
- a dose modulation;
- a scan planning parameter; and
- a reconstruction parameter.

12. The system (100) according to any one of the above claims, wherein the machine learning algorithm is a convolutional neural network (200).

13. A workstation or an imaging system comprising the system according to any one of claims 1 to 12.

14. A computer implemented method (400) for selecting an acquisition parameter for an imaging system, wherein the acquisition parameter at least in part defines an imaging configuration of the imaging system during an imaging procedure with a patient, comprising:
- accessing (410) a depth-related map which is generated on the basis of sensor data from a camera system, wherein the camera system has a field of view which includes at least part of a field of view of the imaging system, wherein the sensor data is obtained before the imaging procedure with the patient and indicative of a distance that different parts of the patient's exterior have towards the camera system;
- applying (420) a machine learning algorithm to the depth-related map to identify the acquisition parameter, wherein:
- the machine learning algorithm is represented by algorithm data which is stored in the memory and accessed by the processor, and
- the machine learning algorithm is trained using training data comprising sets of i) an example depth-related map and ii) as prediction value, an example acquisition, wherein the example acquisition parameter represents a selection by a human operator for use in a previous imaging procedure with a previous patient, wherein the example depth-related map is generated on the basis of sensor data obtained by a previous camera system during the previous imaging procedure; and
- outputting (430) the acquisition parameter to be used by the imaging system.

15. A computer readable medium (500) comprising transitory or non-transitory data (510) representing instructions arranged to cause a processor system to perform the method according to claim 14.

## Patentansprüche

1. System (100) zum Auswählen eines Erfassungsparameters für ein Bildgebungssystem (15), wobei der Erfassungsparameter zumindest teilweise eine Bildgebungskonfiguration des Bildgebungssystems während einer Bildgebungsprozedur mit einem Patienten definiert, umfassend:
- eine Kameradatenschnittstelle (120), die dazu konfiguriert ist, auf eine tiefenbezogene Karte zuzugreifen, die auf Grundlage von Sensordaten von einem Kamerasystem erzeugt wird, wobei das Kamerasystem ein Sichtfeld hat, das zumindest einen Teil eines Sichtfeldes des Abbildungssystems umfasst, wobei die Sensordaten vor dem Abbildungsverfahren mit dem Patienten erfasst werden und einen Abstand anzeigen, den Teile des Äußeren des Patienten zum Kamerasystem haben;
- einen Speicher (140) mit Befehlsdaten, die einen Befehlssatz repräsentieren;
- einen Prozessor (160), der dazu konfiguriert ist, mit der Kameradatenschnittstelle (120) und dem Speicher (160) zu kommunizieren und den Befehlssatz auszuführen, wobei der Befehlssatz, wenn er von dem Prozessor ausgeführt wird, den Prozessor dazu veranlasst, einen maschinellen Lernalgorithmus auf die tiefenbezogene Abbildung anzuwenden, um den Erfassungsparameter zu identifizieren, wobei
- der maschinelle Lernalgorithmus durch Algorithmusdaten repräsentiert wird, die im Speicher gespeichert werden und auf die der Prozessor zugreift, und
- der maschinelle Lernalgorithmus unter Verwendung von Trainingsdaten trainiert wird, die Sätze von i) einer beispielhaften tiefenbezogenen Karte und ii) als Vorhersagewert einen beispielhaften Erfassungsparameter umfassen, wobei der beispielhafte Erfassungsparameter eine Auswahl durch einen menschlichen Bediener in einem vorherigen Bildgebungsverfahren mit einem vorherigen Patienten repräsentiert, wobei die beispielhafte tiefenbezogene Karte auf der Grundlage von Sensordaten erzeugt wird, die durch einen gleichen oder ähnlichen Typ von Kamerasystem während des vorherigen Bildgebungsverfahrens erfasst wurden; und
- eine Ausgabeschnittstelle (170), die dazu konfiguriert ist, die vom Bildgebungssystem zu verwendenden Erfassungsparameter auszugeben.

2. System (100) nach Anspruch 1, das ferner eine Patientendaten-Schnittstelle (60) umfasst, die dazu konfiguriert ist, auf nichtbildliche Patientendaten des Patienten zuzugreifen, und wobei:
- die Trainingsdaten ferner für einen gegebenen Satz von Trainingsdaten beispielsweise nichtbildliche Patientendaten umfassen, die vom gleichen oder ähnlichen Typ wie die nichtbildlichen Patientendaten des Patienten sind; und
- der Satz von Anweisungen, wenn er vom Prozessor (160) ausgeführt wird, bewirkt, dass der Prozessor (160) die nichtbildlichen Patientendaten als zusätzlichen Input für den maschinellen Lernalgorithmus verwendet.

3. System (100) nach Anspruch 2, wobei die Patientendaten-Schnittstelle (60) dazu konfiguriert ist, auf die nichtbildlichen Patientendaten aus einer elektronischen Gesundheitsakte des Patienten zuzugreifen.

4. System (100) nach Anspruch 2 oder 3, bei dem die nichtbildlichen Patientendaten des Patienten mindestens eine der folgenden Angaben umfassen:
- Gewicht des Patienten;
- Alter des Patienten;
- Geschlecht des Patienten;
- Quantifizierung der Fitness des Patienten;
- eine dem Patienten zugeordnete Krankheitsdiagnose;
- eine dem Patienten zugeordnete Medikationsdatei; und
- eine dem Patienten zugeordnete Vitalparameterdatei.

5. System (100) nach einem der oben genannten Ansprüche, wobei:
- die Trainingsdaten ferner für einen gegebenen Satz von Trainingsdaten Beispielgeometriedaten umfassen, die auf eine vorherige relative Geometrie zwischen dem Kamerasystem (18) und dem Abbildungssystem (15) während des vorherigen Abbildungsverfahrens hindeuten; und
- der Befehlssatz, wenn er von dem Prozessor (160) ausgeführt wird, den Prozessor (160) veranlasst, eine aktuelle relative Geometrie zwischen dem Kamerasystem (18) und dem Abbildungssystem (15) in dem Abbildungs- verfahren als zusätzlichen Input für den maschinellen Lernalgorithmus zu verwenden.

6. System (100) nach einem der Ansprüche 1 bis 4, wobei:
- die beispielhafte tiefenbezogene Karte eines gegebenen Satzes der Trainingsdaten auf Grundlage einer vorherigen relativen Geometrie zwischen Kamerasystem (18) und Abbildungssystem (15) erzeugt wird;
- der Befehlssatz, wenn er vom Prozessor (160) ausgeführt werden, den Prozessor (160) dazu veranlasst: eine Abweichung zwischen der vorherigen relativen Geometrie und einer aktuellen relativen Geometrie zwischen dem Kamerasystem (18) und dem Abbildungssystem (15) im Abbildungsverfahren zu bestimmen;
- wenn die Abweichung vorhanden ist oder einen Schwellenwert überschreitet, die tiefenbezogene Karte zu verarbeiten, um die Abweichung vor der besagten Anwendung des maschinellen Lernalgorithmus zu kompensieren.

7. System (100) nach einem der oben genannten Ansprüche, wobei:
- die Kameradatenschnittstelle (120) ferner dazu konfiguriert ist, auf vom Kamerasystem (18) erfasste Bilddaten zuzugreifen, wobei die Bilddaten das Äußere des Patienten zeigen;
- die Trainingsdaten ferner für einen gegebenen Satz von Trainingsdaten Beispielbilddaten umfassen, die von dem vorherigen Kamerasystem (18) erfasst wurden, das das Äußere des vorherigen Patienten während des vorherigen Bildgebungsverfahrens zeigt; und
- der Satz von Anweisungen, wenn er vom Prozessor (160) ausgeführt wird, bewirkt, dass der Prozessor (160) die Bilddaten als zusätzliche Eingabe für den maschinellen Lernalgorithmus verwendet.

8. System (100) nach einem der oben genannten Ansprüche, wobei das System (100) das Kamerasystem (18) umfasst und wobei das Kamerasystem (18) mindestens eines der Folgenden umfasst:
- eine Laufzeitkamera;
- eine LiDAR-Kamera (Light Detection and Ranging);
- eine LaDAR-Kamera (Laser Detection and Ranging);
- eine Stereokamera oder zwei als Stereokamera angeordnete Kameras und
- einen Projektor, der dazu konfiguriert ist, ein bekanntes Muster auf die Außenseite des Patienten zu projizieren und dadurch ein verformtes Muster zu erzeugen, und eine Kamera, die dazu konfiguriert ist, das verformte Muster aufzunehmen.

9. System (100) nach einem der oben genannten Ansprüche, wobei das Bildgebungssystem (15) ein Magnetresonanz-Bildgebungssystem ist, und wobei der Erfassungsparameter einer der folgenden ist:
- ein Parameter, der die Patientenposition in Bezug auf das Magnetresonanz-Bildgebungssystem angibt;
- einen Geometrie-Erfassungsparameter;
- ein Auswahlparameter für ein voreingestelltes Protokoll;
- ein SENSE-Faktor;
- eine SENSE-Richtung.

10. System (100) nach einem der oben genannten Ansprüche, wobei das Abbildungssystem (15) ein Röntgenabbildungssystem ist und wobei der Erfassungsparameter einer der folgenden ist:
- eine Röhrenspannung;
- ein Röhrenstrom;
- ein Gitter;
- ein Kollimationsfenster; und
- ein Geometrieparameter eines Kollimators.

11. System (100) gemäß einem der oben genannten Ansprüche, wobei das Abbildungssystem (15) ein Computertomographie-Abbildungssystem ist und wobei der Erfassungsparameter einer der folgenden ist:
- ein Stromversorgungspegel;
- ein Röhrenstrom,
- eine Dosis-Modulation;
- ein Scan-Planungsparameter und
- ein Rekonstruktionsparameter.

12. System (100) nach einem der oben genannten Ansprüche, wobei der maschinelle Lernalgorithmus ein Convolutional Neural Network (200) ist.

13. Arbeitsstation oder Bildgebungssystem, das das System nach einem der Ansprüche 1 bis 12 umfasst.

14. Computerimplementiertes Verfahren (400) zum Auswählen eines Erfassungsparameters für ein Bildgebungssystem, wobei der Erfassungsparameter zumindest teilweise eine Bildgebungskonfiguration des Bildgebungssystems während eines Bildgebungsverfahrens mit einem Patienten definiert, umfassend:
- Zugriff (410) auf eine tiefenbezogene Karte, die auf der Grundlage von Sensordaten von einem Kamerasystem erzeugt wird, wobei das Kamerasystem ein Sichtfeld hat, das zumindest einen Teil eines Sichtfeldes des Abbildungssystems umfasst, wobei die Sensordaten vor dem Abbildungsverfahren mit dem Patienten empfangen werden und einen Abstand anzeigen, den verschiedene Teile des Äußeren des Patienten zum Kamerasystem haben;
- Anwendung (420) eines maschinellen Lernalgorithmus auf die tiefenbezogene Karte, um den Erfassungsparameter zu identifizieren, wobei:
- der maschinelle Lernalgorithmus durch Algorithmusdaten repräsentiert wird, die im Speicher gespeichert werden und auf die der Prozessor zugreift, und
- der maschinelle Lernalgorithmus anhand von Trainingsdaten trainiert wird, die Folgendes umfassen: Sätze von i) einer beispielhaften tiefenbezogenen Karte und ii) als Vorhersagewert eine Beispielaufnahme, wobei der Beispielaufnahmeparameter eine Auswahl durch einen menschlichen Operator zur Verwendung in einem vorherigen Bildgebungsverfahren mit einem vorherigen Patienten darstellt, wobei die beispielhafte tiefenbezogene Karte auf Grundlage von Sensordaten erzeugt wird, die von einem vorherigen Kamerasystem während des vorherigen Bildgebungsverfahrens erfasst wurden; und
- Ausgabe (430) des vom Bildgebungssystem zu verwendenden Aufnahmeparameters.

15. Computerlesbares Medium (500) mit vorübergehenden oder nicht vorübergehenden Daten (510), die Befehle darstellen, die so ausgelegt sind, dass sie ein Prozessorsystem veranlassen, das Verfahren nach Anspruch 14 auszuführen.

## Revendications

1. Système (100) de sélection d'un paramètre d'acquisition pour un système d'imagerie (15), dans lequel le paramètre d'acquisition définit au moins en partie une configuration d'imagerie du système d'imagerie au cours d'une procédure d'imagerie avec un patient, comprenant :
- une interface de données de caméra (120) configurée pour accéder à une carte de profondeur générée sur la base des données de capteur à partir d'un système de caméra, dans laquelle le système de caméra a un champ de vision comprenant au moins une partie d'un champ de vision du système d'imagerie, dans laquelle les données de capteur sont obtenues avant la procédure d'imagerie avec le patient et représentent la distance existante des parties de l'extérieur d'un patient vis-à-vis du système de caméra ;
- une mémoire (140) comprenant des données d'instruction représentant une série d'instructions ;
- un processeur (160) configuré pour communiquer avec l'interface de données de la caméra (120) et la mémoire (160) et pour exécuter la série d'instructions, dans lequel la série d'instructions, une fois exécutées par le processeur, permettent au processeur d'appliquer un algorithme d'apprentissage machine à la carte de profondeur pour identifier le paramètre d'acquisition, dans lequel :
- l'algorithme d'apprentissage est représenté par des données d'algorithme stockées dans la mémoire et accessibles par le processeur, et
- l'algorithme d'apprentissage machine est configuré pour utiliser des données d'apprentissage comprenant des séries d'une i) carte de profondeur illustrative et ii) en tant que valeur de prédiction, un paramètre d'acquisition illustratif, dans lequel le paramètre d'acquisition illustratif représente un choix réalisé par un opérateur humain dans une procédure d'imagerie précédente avec un patient précédent, dans lequel la carte de profondeur illustrative est générée sur la base des données de capteur obtenues par un même type ou un type similaire de système de caméra au cours de la procédure d'imagerie précédente ; et
- une interface de sortie (170) configurée pour extraire le paramètre d'acquisition devant être utilisé par le système d'imagerie.

2. Système (100) selon la revendication 1, comprenant en outre une interface de données de patient (60) configurée pour accéder aux données non-image patient du patient, et dans lequel :
- les données d'apprentissage comprennent en outre, pour une série spécifique de données d'apprentissage, des données non-image du patient illustratives qui sont du même type ou d'un type similaire aux données non-image patient du patient ; et
- la série d'instructions, une fois exécutées par le processeur (160), permettent au processeur (160) d'utiliser les données non-image du patient comme entrée supplémentaire pour l'algorithme d'apprentissage machine.

3. Système (100) selon la revendication 2, dans lequel l'interface de données patient (60) est configurée pour accéder aux données non-image patient à partir d'un dossier médical électronique du patient.

4. Système (100) selon la revendication 2 ou 3, dans lequel les données non-image patient du patient comprennent au moins :
- un poids du patient ;
- un âge du patient ;
- un sexe du patient ;
- une quantification d'un niveau de forme du patient ;
- un diagnostic de maladie associé au patient ;
- un dossier sur les traitements administrés au patient ; et
- un dossier sur les paramètres vitaux du patient.

5. Système (100) selon l'une quelconque des revendications ci-dessus, dans lequel :
- les données d'apprentissage comprennent en outre, pour une série spécifique de données d'apprentissage, des données de géométrie illustratives représentatives d'une géométrie relative précédente entre le système de caméra (18) et le système d'imagerie (15) au cours de la procédure d'imagerie précédente ; et
- la série d'instructions, une fois exécutées par le processeur (160), permettent au processeur (160) d'utiliser une géométrie relative actuelle entre le système de caméra (18) et le système d'imagerie (15) dans la procédure d'imagerie comme entrée supplémentaire de l'algorithme d'apprentissage machine.

6. Système (100) selon l'une quelconque des revendications 1 à 4, dans lequel :
- la carte de profondeur illustrative d'une série spécifique de données d'apprentissage est générée sur la base d'une géométrie relative précédente entre le système de caméra (18) et le système d'imagerie (15) ;
- la série d'instructions, une fois exécutées par le processeur (160), permettent au processeur (160) de :
- déterminer un écart entre la géométrie relative précédente et une géométrie relative actuelle entre le système de caméra (18) et le système d'imagerie (15) dans la procédure d'imagerie ;
- si l'écart existe ou dépasse un seuil, traiter la carte de profondeur pour compenser l'écart avant ladite application de l'algorithme d'apprentissage machine.

7. Système (100) selon l'une quelconque des revendications ci-dessus, dans lequel :
- l'interface de données de caméra (120) est en outre configurée pour accéder aux données image acquises par le système de caméra (18), dans lequel les données image montrent l'extérieur du patient ;
- les données d'apprentissage comprennent en outre, pour une série spécifique de données d'apprentissage, des données image illustratives acquises par le système de caméra précédent (18) qui montre l'extérieur précédent du patient au cours de la procédure d'imagerie précédente ; et
- la série d'instructions, une fois exécutées par le processeur (160), permettent au processeur (160) d'utiliser les données image comme entrée supplémentaire de l'algorithme d'apprentissage machine.

8. Système (100) selon l'une quelconque des revendications ci-dessus, dans lequel le système (100) comprend le système de caméra (18), et dans lequel le système de caméra (18) comprend au moins :
- une caméra de temps de vol ;
- une caméra de détection et d'estimation de la distance par la lumière (LiDAR) ;
- une caméra de détection et d'estimation de la distance par laser (LaDAR) ;
- une caméra stéréo, ou deux caméras agencées en tant que caméras stéréo ; et
- un projecteur configuré pour projeter une configuration connue sur l'extérieur du patient, donnant ainsi une configuration déformée, et une caméra configurée pour enregistrer la configuration déformée.

9. Système (100) selon l'une quelconque des revendications ci-dessus, dans lequel le système d'imagerie (15) est un système d'imagerie à résonance magnétique, et dans lequel le paramètre d'acquisition figure parmi :
- un paramètre spécifiant le positionnement du patient par rapport au système d'imagerie à résonance magnétique ;
- un paramètre d'acquisition de géométrie ;
- un paramètre de sélection pour un protocole prédéfini ;
- un facteur SENSE ;
- une direction SENSE.

10. Système (100) selon l'une quelconque des revendications ci-dessus, dans lequel le système d'imagerie (15) est un système d'imagerie radiographique, et dans lequel le paramètre d'acquisition figure parmi :
- une tension de tube ;
- un courant de tube ;
- une grille ;
- une fenêtre de collimation ; et
- un paramètre de géométrie d'un collimateur ;

11. Système (100) selon l'une quelconque des revendications ci-dessus, dans lequel le système d'imagerie (15) est un système d'imagerie de tomodensitométrie, et dans lequel le paramètre d'acquisition figure parmi :
- un niveau de puissance électrique ;
- un courant de tube ;
- une modulation de dose ;
- un paramètre de planification de balayage ; et
- un paramètre de reconstruction.

12. Système (100) selon l'une quelconque des revendications ci-dessus, dans lequel l'algorithme d'apprentissage machine est un réseau neuronal convolutionnel (200).

13. Station de travail ou système d'imagerie comprenant le système selon l'une quelconque des revendications 1 à 12.

14. Procédé mis en œuvre par ordinateur (400) pour sélectionner un paramètre d'acquisition pour un système d'imagerie, dans lequel le paramètre d'acquisition définit au moins en partie une configuration d'imagerie du système d'imagerie au cours d'une procédure d'imagerie avec un patient, comprenant :
- l'accès (410) à une carte de profondeur générée sur la base des données de capteur d'un système de caméra, dans lequel le système de caméra a un champ de vision qui comprend au moins une partie d'un champ de vision du système d'imagerie, dans lequel les données de capteur sont obtenues avant la procédure d'imagerie avec le patient et représentent la distance existante des différentes parties de l'extérieur du patient vis-à-vis du système de caméra ;
- l'application (420) d'un algorithme d'apprentissage machine à la carte de profondeur pour identifier le paramètre d'acquisition, dans lequel ;
- l'algorithme d'apprentissage machine est représenté par les données d'algorithme qui sont stockées dans la mémoire et accessibles par le processeur, et
- l'algorithme d'apprentissage machine est configuré pour utiliser les données d'apprentissage comprenant des séries d'une i) carte de profondeur illustrative et ii) en tant que valeur de prédiction, une acquisition illustrative, dans lequel le paramètre d'acquisition illustratif représente une sélection par un opérateur humain pour utilisation dans une procédure d'imagerie précédente avec un précédent patient, dans lequel la carte de profondeur est générée sur la base des données de capteur obtenues par un système de caméra précédent au cours de la procédure d'imagerie précédente ; et
- l'extraction (430) du paramètre d'acquisition devant être utilisé par le système d'imagerie.

15. Support lisible par ordinateur (500) comprenant des données transitoires et non transitoires (510) représentant des instructions agencées de façon à permettre à un système de processeur de réaliser le procédé selon la revendication 14.
